# FASCICULE DE BREVET EUROPEEN

(11) **EP 1 532 967 B1**
(45) Date de publication et mention de la délivrance du brevet: **13.03.2013**
(21) Numéro de dépôt: 04292657.6
(22) Date de dépôt: 10.11.2004
(51) Int. Cl.: A61K 8/73, A61Q 5/06

(54) **Composition cosmétique comprenant de la gomme de gellane ou un de ses derivés, un composé solide et un sel monovalent, procédés mettant en oeuvre cette composition et utilisations**
Kosmetische Zusammensetzung enthaltend Gellangummi oder ein Derivat davon, ein Feststoff und ein Alkohol, Verfahren auf Basis dieser Zusammensetzung und Anwendungen
Cosmetic composition comprising gellan gum or a derivative thereof, a solid compound and an alcohol, processes using this composition and uses thereof

(30) Priorité: 18.11.2003 FR 0313488
(43) Date de publication de la demande: 25.05.2005
(73) Titulaire: L'Oréal, 75008 Paris (FR)
(72) Inventeur: Laurent, Ludivine, 92400 Courbevoie (FR)
(74) Mandataire: Dossmann, Gérard

(56) Documents cités:
- EP-A- 0 985 410
- WO-A-01/28503
- GB-A- 2 384 705
- US-A- 5 523 078
- US-A- 6 110 473
- US-A1- 2003 033 678
- US-A1- 2003 072 779
- US-B1- 6 391 288
- "Base composition for medicines, food and cosmetics, containing matrix composed of gellan gum, cations and water" WPI WORLD PATENT INF, 1997, XP002125782
- TANG J ET AL: "Compression strength and deformation of gellan gels formed with mono- and divalent cations" CARBOHYDRATE POLYMERS, APPLIED SCIENCE PUBLISHERS, LTD. BARKING, GB, vol. 29, no. 1, 1996, pages 11-16, XP004034348 ISSN: 0144-8617
- EDWARD P DULIBA ET AL: "Clarified high acyl high molecular weight gellan gum applications" RESEARCH DISCLOSURE, KENNETH MASON PUBLICATIONS, HAMPSHIRE, GB, vol. 463, no. 7, novembre 2002 (2002-11), XP007131518 ISSN: 0374-4353
- JOHN SWAZEY: "Gellan gum in toothpaste" RESEARCH DISCLOSURE, KENNETH MASON PUBLICATIONS, HAMPSHIRE, GB, vol. 440, no. 130, décembre 2000 (2000-12), XP007127315 ISSN: 0374-4353

## Description

La présente demande a pour objet une composition cosmétique liquide, aqueuse, non lavante comprenant, dans un milieu cosmétiquement acceptable, au moins une gomme de gellane ou un de ses dérivés, au moins un composé solide et au moins un sel monovalent. La présente demande concerne encore un procédé pour la mise en forme ou le maintien de la coiffure dans lequel cette composition cosmétique est mise en oeuvre, ainsi que les utilisations de cette composition.

Dans le domaine de la cosmétique, il est courant d'utiliser des compositions comprenant des particules solides. Récemment, une mode consistant à ajouter des composés irisants ou nacrants dans les compositions cosmétiques a généré une recrudescence de ces compositions sur le marché. Ainsi sont apparus non seulement des produits de maquillage de la peau et des phanères mais également des compositions lavantes pour le corps, des shampooings, des compositions pour mettre en forme les cheveux comprenant des particules solides en suspension.

Le principal problème posé par ces compositions est leur stabilité. La stabilité des suspensions de particules solides est liée à la viscosité du support d'une part et au maintien de l'homogénéité de la répartition des particules solides dans le support d'autre part. Suite à une élévation de la température ou simplement au cours du temps, il est courant d'assister à une sédimentation des particules solides (lorsque la densité des particules solides est supérieure à la densité du support) ou à un crémage (lorsque la densité des particules solides est inférieure à la densité du support).

Pour obtenir des compositions stables, toutes sortes d'agents gélifiants ont été utilisés, parmi ceux-ci on compte les polymères ou copolymères d'acides organiques carboxyliques insaturés ou d'esters insaturés, les dérivés polysaccharidiques, les gommes, les silicates colloïdaux, les polyéthylèneglycols, les polyvinylpyrrolidones, les gels de silice hydrophiles. Cependant, les résultats obtenus avec ces agents gélifiants ne sont pas toujours satisfaisants et peuvent varier avec la nature des particules solides utilisées.

Dans la demande FR 2 700 952 de la Demanderesse, il a été proposé de stabiliser des gels comprenant éventuellement des particules solides en suspension au moyen de microsphères creuses, expansées, en matériau thermoplastique, ayant une masse spécifique de 15 à 200 kg/m³, ces microsphères étant ajoutées à la composition à raison de 0,1 à 10 % en poids de microsphères par rapport au poids total de la composition, ladite composition étant en outre exemple de matière grasse.

La demande WO 02/072063 déposée par la société Beiersdorf A.G. décrit des compositions lavantes comprenant des particules en suspension dans un mélange de tensio-actifs, de gomme de xanthane et de gomme de gellane.

La demande EP 0 985 410 décrit une composition cosmétique ou pharmaceutique destinée à être appliquée sur la peau, et qui comprend au moins un polymère carboxyvinylique et de la gomme de gellane.

US 5 523 078 A (BAYLIN MICHAEL E) 4 juin 1996 (1996-06-04) divulgue (rev. 1) une composition capillaire liquide, aqueuse, non lavante (moins de 4% de solubilisant) comprenant:
- 0.1-1% de gomme de gellane
- un sel de cation monovalent (Na₂EDTA).

De manière surprenante est avantageuse, la demanderesse a découvert que l'utilisation d'une combinaison d'une gomme de gellane ou un de ses dérivés et d'un sel monovalent permet de stabiliser les compositions liquides, aqueuses, non lavantes, contenant des particules solides d'agar-agar.

Dans la présente demande les expressions « particules solides » et « composés solides » sont équivalentes et font référence à des composés ou particules qui sont toujours à l'état solide dans la composition cosmétique finale

La combinaison d'une gomme de gellane ou un de ses dérivés et d'un sel monovale permet une parfaite mise en suspension des particules solides d'agar-agar même dans un milieu présentant une très faible viscosité (de l'ordre de 1 à 10 poises soit 0,1 à 1 Pa.s). La composition cosmétique obtenue est stable pendant plusieurs mois même lorsqu'elle est soumise à des variations de température.

Le caractère thixotrope des compositions comprenant cette combinaison permet une restitution parfaite des propriétés de ces compositions cosmétiques coiffantes lorsqu'elles sont appliquées au moyen d'un spray ou en aérosol.

La combinaison est utilisée dans des compositions cosmétiques liquides, aqueuses, non lavantes. Avantageusement, elle est utilisée dans des compositions de mise en forme ou de maintien des cheveux.

Un autre avantage de cette combinaison est qu'elle permet l'obtention de compositions qui confèrent aux cheveux de bonnes propriétés cosmétiques notamment en termes de toucher (toucher doux) et de démêlage.

Lorsque la combinaison est utilisée dans des compositions cosmétiques pour la mise en forme et/ou le maintien de la coiffure, les compositions obtenues permettent une bonne fixation et un bon maintien des cheveux c'est-à-dire un effet coiffant qui persiste tout au long de la journée, voire plusieurs jours, qui présente une bonne tenue à l'humidité et qui s'élimine facilement au shampooing.

La présente invention concerne l'utilisation d'une combinaison comprenant une gomme de gellane ou un de ses dérivés et d'un sel monovalent en coiffage et l'utilisation d'une combinaison constituée d'une gomme de gellane ou un de ses dérivés et d'un sel monovalent en coiffage, selon cette dernière réalisation, la combinaison ne contient aucun agent fixant classique.

La présente invention a pour objet une composition cosmétique liquide, aqueuse, non lavante, contenant, dans un milieu cosmétiquement acceptable au moins une gomme de gellane ou un de ses dérivés, au moins un composé solide choisi parmi les sphères d'agar-agar et au moins un sel monovalent.

De préférence la composition cosmétique selon l'invention est une composition cosmétique capillaire, de manière préférée une composition cosmétique capillaire coiffante

Un autre objet de la présente invention consiste en un procédé pour la mise en forme ou le maintien de la coiffure dans lequel la composition cosmétique selon l'invention est mise en oeuvre.

Un quatrième objet de l'invention concerne les utilisations de cette composition cosmétique en tant que composition coiffante pour la fixation et le maintien des cheveux, composition de soin des cheveux, composition de conditionnement des cheveux notamment pour apporter de la douceur aux cheveux, ou encore composition de maquillage des cheveux ou des phanères (cils, ongles, poils).

Ainsi, la présente demande concerne l'utilisation de la composition selon l'invention pour apporter de la fixation aux cheveux, pour apporter du maintien aux cheveux, pour apporter de la cosmétique aux cheveux.

La composition cosmétique selon l'invention peut se présenter sous la forme d'un spray, d'une mousse ou d'un gel.

D'autres objets, caractéristiques, aspects et avantages de l'invention apparaîtront encore plus clairement à la lecture de la description et des exemples qui suivent.

Sans vouloir être lié à une quelconque théorie, les compositions cosmétiques selon la présente demande peuvent se présenter sous forme de gels, c'est-à-dire un réseau tridimensionnel de molécules qui retient dans ses mailles une quantité importante de solvant. La formation d'un tel réseau constitue sa gélification.

Les compositions cosmétiques selon la présente demande peuvent aussi se présenter sous forme de mousses.

Par « composition cosmétique coiffante » au sens de la présente demande, on entend une composition pour la mise en forme ou le maintien de la coiffure.

Par « composition liquide » selon la présente demande, on entend que la viscosité de la composition est comprise entre la viscosité de l'eau et 50 poises, de préférence entre la viscosité de l'eau et 20 poises.

Par « composition aqueuse » selon la présente demande, on entend que le milieu cosmétiquement acceptable utilisé dans les compositions selon la présente invention est un milieu aqueux contenant éventuellement au moins un solvant organique additionnel.

Le solvant organique additionnel utilisé dans les compositions selon la présente invention est choisi parmi les alcools inférieurs en C₁-C₄ comme l'éthanol, l'isopropanol, le tertiobutanol, le n-butanol, les polyols comme le propylèneglycol, les éthers de polyols et leurs mélanges, de préférence l'alcool utilisé est l'éthanol.

La composition aqueuse selon la présente demande contient moins de 10% en poids de composés huileux, par « composés huileux », on entend des composés huileux aussi bien liquides que solides.

Les compositions selon l'invention sont des compositions cosmétiques non lavantes, par « composition cosmétique non lavante » au sens de la présente demande, on entend que la composition ne contient pas plus de 4% en poids d'agents tensio-actifs par rapport au poids total de la composition.

La gomme de gellane est un polysaccharide produit par fermentation aérobie de *Sphingomonas elodea,* plus communément nommé *Pseudomonas elodea.* Ce polysaccharide linéaire est constitué par l'enchaînement des monosaccharides suivants : D-Glucose, acide D-glucuronique et L-Rhamnose. A l'état natif, la gomme de gellane est hautement acylée.

La gomme de gellane utilisée de manière préférée dans les compositions selon la présente invention est une gomme de gellane au moins partiellement déacylée. Cette gomme de gellane au moins partiellement déacylée est obtenue par un traitement alcalin à haute température.

On utilisera par exemple une solution de KOH ou de NaOH.

La gomme de gellane purifiée vendue sous la dénomination commerciale « KELCOGEL® » par la société KELCO convient pour préparer les compositions selon l'invention.

Les dérivés de la gomme de gellane sont tous les produits obtenus en mettant en oeuvre des réactions chimiques classiques, tels que notamment les estérifications, addition d'un sel d'un acide organique ou minéral.

A titre de dérivé de la gomme de gellane, on utilise par exemple la gomme de welane. La gomme de welane est une gomme de gellane modifiée par fermentation au moyen de *Alcaligenes* souche ATCC 31 555. La gomme de welane possède une structure pentasaccharidique récurrente formée d'une chaîne principale constituée d'unités D-Glucose, acide D-glucuronique et L-Rhamnose sur laquelle un motif pendant de L-Rhamnose ou de L-Mannose est greffé.

La gomme de welane vendue sous la dénomination commerciale « KELCO CRETE ®» par la société KELCO convient pour préparer les compositions selon l'invention.

La concentration en gomme de gellane ou ses dérivés utilisée dans les compositions selon la présente invention est comprise entre 0,005 et 10%, de préférence entre 0,01 et 5% et de manière encore plus préférée entre 0,02 et 3% en poids par rapport au poids total de la composition.

Les sels monovalents utilisables dans les compositions selon la présente invention sont les sels de cations monovalents tels que les sels des métaux alcalins, les sels d'ammonium, les sels d'amines organiques ou encore leurs mélanges. Les cations monovalents des métaux alcalins sont les cations suivants : Li⁺, Na⁺, K⁺, Rb⁺, Cs⁺, Fr⁺. Dans les compositions selon la présente invention on utilise de préférence le Na⁺.

Le contre-ion est un anion minéral ou organique, de préférence le contre-ion est Cl⁻.

De préférence, le sel utilisé est NaCl.

La concentration en sel(s) monovalent(s) utilisée dans les compositions selon la présente invention est comprise entre 0,01 et 10%, de préférence entre 0,05 et 5% en poids par rapport au poids total de la composition.

Avantageusement, le rapport sel monovalent /gomme de gellane ou dérivé est compris entre 1 et 50% et de préférence entre 2 et 30%.

Les composés ou particules solides utilisables dans les compositions cosmétiques selon la présente demande sont les composés cosmétiquement acceptables qui sont solides dans des conditions de température ambiante (entre 20°C et 40°C) et de pression atmosphérique. Les sphères d'agar-agar présentant une solubilité dans le milieu cosmétiquement acceptable inférieure à 0,01g/100g peuvent être utilisées. En particulier les sphères creuses présentant la solubilité requise peuvent être utilisées.

De préférence les composés solides ne sont pas sous forme de capsules contenant un principe actif.

La concentration en composés solides utilisée dans les compositions selon la présente invention est comprise entre 0,001 et 5%, de préférence entre 0,01 et 2% en poids par rapport au poids total de la composition.

La composition selon l'invention peut contenir en outre au moins un adjuvant additionnel qui n'est pas un composé solide tel qu'utilisé dans les compositions selon la présente demande. Cet adjuvant additionnel peut notamment être choisi parmi les silicones sous forme soluble, dispersée, micro-dispersée, les agents tensio-actifs non-ioniques, anioniques, cationiques et amphotères, les polymères non-ioniques, anioniques, cationiques et amphotères, les céramides et pseudo-céramides, les vitamines et pro-vitamines dont le panthénol, les huiles végétales, animales, minérales et synthétiques, les cires autres que les céramides et pseudo-céramides, les filtres solaires hydrosolubles et liposolubles, liquides ou solides, siliconés ou non silicones, les colorants, les agents séquestrants, les agents plastifiants, les agents solubilisants, les agents acidifiants, des agents alcalinisants, les agents neutralisants, les agents épaississants minéraux et organiques, les agents anti-oxydants, les hydroxyacides, les glycols, les agents de pénétration, les parfums et les agents conservateurs.

L'homme de métier veillera à choisir les éventuels additifs et leur quantité de manière à ce qu'ils ne nuisent pas aux propriétés des compositions selon la présente invention.

Ces additifs sont présents dans la composition selon l'invention en une quantité allant de 0 à 20 % en poids par rapport au poids total de la composition.

Les exemples suivants illustrent la présente invention et ne doivent être considérés en aucune manière comme limitant l'invention.

### EXEMPLES

On prépare les compositions formulées en spray en flacon pompe dont les teneurs des différents constituants en g/100g sont les suivantes :

### Composition 1

| Constituant | g/100g |
|---|---|
| Gomme de gellane | 0,04 |
| NaCl | 1,0 |
| sphères d'agar-agar | 0,15 |
| Parfum | qs |
| Eau distillée | Qsp 100 |

Cette composition présente une viscosité proche de celle de l'eau. Les particules solides restent en suspension. Après 2 mois de conservation à 45°C, on n'observe aucun changement de l'aspect de cette composition.

## Revendications

1. Utilisation en tant que composition cosmétique capillaire d'une composition cosmétique liquide, aqueuse, non lavante, contenant, dans un milieu cosmétiquement acceptable,
■ au moins une gomme de gellane ou un de ses dérivés,
■ au moins un composé solide choisi parmi les sphères d'agar-agar en une concentration comprise entre 0,001% et 5% en poids par rapport au poids total de la composition,
■ et au moins un sel monovalent choisi parmi les sels de cations monovalents,
ladite composition étant non lavante, elle ne contient pas plus de 4 % en poids d'agents tensio-actifs par rapport au poids total de la composition.

2. Utilisation selon la revendication 1 telle que le dérivé de gomme de gellane est une gomme de welane.

3. Utilisation selon l'une des revendications précédentes telle que la concentration en gomme de gellane ou ses dérivés est comprise entre 0,005 % et 10%, de préférence 0,01% et 5% et de manière encore plus préférée entre 0,02 et 3% en poids par rapport au poids total de la composition.

4. Utilisation selon l'une des revendications précédentes telle que le sel monovalent est choisi parmi les sels des métaux alcalins, les sels d'ammonium, les sels d'amines organiques ou encore leurs mélanges.

5. Utilisation selon la revendication 4 telle que le sel monovalent de métal alcalin est un sel de Na⁺.

6. Utilisation selon l'une des revendications précédentes telle que la concentration en sel monovalent est comprise entre 0,01 % et 10%, de préférence entre 0,05% et 5% en poids par rapport au poids total de la composition.

7. Utilisation selon l'une des revendications précédentes telle que la composition contient au moins un adjuvant additionnel choisi parmi les silicones sous forme soluble, dispersée, micro-dispersée, les agents tensio-actifs non-ioniques, anioniques, cationiques et amphotères, les polymères non-ioniques, anioniques, cationiques et amphotères, les céramides et pseudo-céramides, les vitamines et pro-vitamines dont le panthénol, les huiles végétales, animales, minérales et synthétiques, les cires autres que les céramides et pseudo-céramides, les filtres solaires hydrosolubles et liposolubles, liquides ou solides, siliconés ou non siliconés, les colorants, les agents séquestrants, les agents plastifiants, les agents solubilisants, les agents acidifiants, des agents alcalinisants, les agents neutralisants, les agents épaississants minéraux et organiques, les agents anti-oxydants, les hydroxyacides, les glycols, les agents de pénétration, les parfums et les agents conservateurs.

8. Utilisation selon l'une des revendications précédentes telle que la composition se présente sous la forme d'un gel.

9. Utilisation selon l'une des revendications 1 à 7 telle que la composition se présente sous la forme d'une mousse.

10. Utilisation selon l'une des revendications 1 à 7 telle que la composition se présente sous la forme d'un spray.

11. Utilisation selon l'une des revendications 1 à 10 en tant que composition coiffante pour la fixation et le maintien des cheveux.

12. Utilisation selon l'une des revendications 1 à 10 en tant que composition de soin des cheveux.

13. Utilisation selon l'une des revendications 1 à 10 en tant que composition de conditionnement des cheveux notamment pour apporter de la douceur aux cheveux.

14. Procédé pour la mise en forme ou le maintien de la coiffure dans lequel est mise en oeuvre une composition liquide, aqueuse, non lavante, contenant, dans un milieu cosmétiquement acceptable,
■ au moins une gomme de gellane ou un de ses dérivés,
■ au moins un composé solide choisi parmi les sphères d'agar-agar en une concentration comprise entre 0,001% et 5% en poids par rapport au poids total de la composition,
■ et au moins un sel monovalent choisi parmi les sels de cations monovalents,
ladite composition étant non lavante, elle ne contient pas plus de 4 % en poids d'agents tensio-actifs par rapport au poids total de la composition.

## Claims

1. Use as a cosmetic hair composition of a non-washing aqueous liquid cosmetic composition containing, in a cosmetically acceptable medium,
• at least one gellan gum or a derivative thereof,
• at least one solid compound chosen from agar spheres in a concentration of between 0.001% and 5% by weight relative to the total weight of the composition,
• and at least one monovalent salt chosen from salts of monovalent cations,
the composition being a non-washing composition, it does not contain more than 4% by weight of surfactants relative to the total weight of the composition.

2. Use according to Claim 1, such that the gellan gum derivative is a welan gum.

3. Use according to either of the preceding claims, such that the concentration of gellan gum or derivatives thereof is between 0.005% and 10%, preferably between 0.01% and 5% and even more preferably between 0.02% and 3% by weight relative to the total weight of the composition.

4. Use according to one of the preceding claims, such that the monovalent salt is chosen from the alkali metal salts, ammonium salts, organic amine salts, or mixtures thereof.

5. Use according to Claim 4, such that the monovalent alkali metal salt is an Na⁺ salt.

6. Use according to one of the preceding claims, such that the concentration of monovalent salt is between 0.01% and 10% and preferably between 0.05% and 5% by weight relative to the total weight of the composition.

7. Use according to one of the preceding claims, such that the composition contains at least one additional adjuvant chosen from silicones in soluble, dispersed or microdispersed form, nonionic, anionic, cationic and amphoteric surfactants, nonionic, anionic, cationic and amphoteric polymers, ceramides and pseudoceramides, vitamins and provitamins, including panthenol, plant, animal, mineral and synthetic oils, waxes other than ceramides and pseudoceramides, silicone or non-silicone, liquid or solid, water-soluble and liposoluble sunscreens, dyes, sequestrants, plasticizers, solubilizers, acidifying agents, basifying agents, neutralizers, mineral and organic thickeners, antioxidants, hydroxy acids, glycols, penetrants, fragrances and preserving agents.

8. Use according to one of the preceding claims, such that the composition is in the form of a gel.

9. Use according to one of Claims 1 to 7, such that the composition is in the form of a mousse.

10. Use according to one of Claims 1 to 7, such that the composition is in the form of a spray.

11. Use according to one of Claims 1 to 10 as a styling composition for fixing and holding the hair.

12. Use according to one of Claims 1 to 10 as a haircare composition.

13. Use according to one of Claims 1 to 10 as a hair conditioning composition, especially for giving the hair softness.

14. Process for shaping or holding the hairstyle, in which a non-washing aqueous liquid composition is used, containing, in a cosmetically acceptable medium,
• at least one gellan gum or a derivative thereof,
• at least one solid compound chosen from agar spheres in a concentration of between 0.001% and 5% by weight relative to the total weight of the composition,
• and at least one monovalent salt chosen from salts of monovalent cations,
the composition being a non-washing composition, it does not contain more than 4% by weight of surfactants relative to the total weight of the composition.

## Patentansprüche

1. Verwendung einer nicht waschaktiven, wässrigen, flüssigen kosmetischen Zusammensetzung, die in einem kosmetisch unbedenklichen Medium
• mindestens ein Gellangummi oder ein Derivat davon,
• mindestens eine feste Verbindung, die aus Agar-Agar-Kugeln ausgewählt ist, in einer Konzentration zwischen 0,001 und 5 Gew.-%, bezogen auf das Gesamtgewicht der Zusammensetzung,
• und mindestens ein einwertiges Salz, das aus den Salzen einwertiger Kationen ausgewählt ist,
enthält, wobei die nicht waschaktive Zusammensetzung nicht mehr als 4 Gew.-% Tenside, bezogen auf das Gesamtgewicht der Zusammensetzung, enthält, als haarkosmetische Zusammensetzung.

2. Verwendung nach Anspruch 1, wobei es sich bei dem Gellangummiderivat um ein Welangummi handelt.

3. Verwendung nach einem der vorhergehenden Ansprüche, wobei die Konzentration an Gellangummi oder Derivaten davon zwischen 0,005 und 10 Gew.-%, vorzugsweise zwischen 0,01 und 5 Gew.-% und weiter bevorzugt zwischen 0,02 und 3 Gew.-%, bezogen auf das Gesamtgewicht der Zusammensetzung, liegt.

4. Verwendung nach einem der vorhergehenden Ansprüche, wobei das einwertige Salz aus Alkalimetallsalzen, Ammoniumsalzen, Salzen von organischen Aminen oder Mischungen davon ausgewählt ist.

5. Verwendung nach Anspruch 4, wobei es sich bei dem einwertigen Alkalimetallsalz um ein Na⁺-Salz handelt.

6. Verwendung nach einem der vorhergehenden Ansprüche, wobei die Konzentration an einwertigem Salz zwischen 0,01 und 10 Gew.-%, vorzugsweise zwischen 0,05 und 5 Gew.-%, bezogen auf das Gesamtgewicht der Zusammensetzung, liegt.

7. Verwendung nach einem der vorhergehenden Ansprüche, wobei die Zusammensetzung mindestens einen zusätzlichen Hilfsstoff, der aus Silikonen in löslicher, dispergierter und mikrodispergierter Form, nichtionischen, anionischen, kationischen und amphoteren Tensiden, nichtionischen, anionischen, kationischen und amphoteren Polymeren, Ceramiden und Pseudoceramiden, Vitaminen und Provitaminen, darunter Panthenol, pflanzlichen, tierischen, mineralischen und synthetischen Ölen, Wachsen, bei denen es sich nicht um Ceramide und Pseudoceramide handelt, wasserlöslichen und fettlöslichen, flüssigen oder festen Silikon- und Nichtsilikon-Lichtschutzmitteln, Farbmitteln, Sequestriermitteln, Weichmachern, Solubilisierungsmitteln, Acidifizierungsmitteln, Alkalinisierungsmitteln, Neutralisationsmitteln, anorganischen und organischen Verdickungsmitteln, Antioxidantien, Hydroxysäuren, Glykolen, Penetriermitteln, Duftstoffen und Konservierungsstoffen ausgewählt ist, enthält.

8. Verwendung nach einem der vorhergehenden Ansprüche, wobei die Zusammensetzung in Form eines Gels vorliegt.

9. Verwendung nach einem der Ansprüche 1 bis 7, wobei die Zusammensetzung in Form eines Schaums vorliegt.

10. Verwendung nach einem der Ansprüche 1 bis 7, wobei die Zusammensetzung in Form eines Sprays vorliegt.

11. Verwendung nach einem der Ansprüche 1 bis 10 als Frisierzusammensetzung zur Fixierung und Festigung der Haare.

12. Verwendung nach einem der Ansprüche 1 bis 10 als Haarpflegezusammensetzung.

13. Verwendung nach einem der Ansprüche 1 bis 10 als Haarkonditionierungszusammensetzung, insbesondere um den Haaren Weicheit zu verleihen.

14. Verfahren zum Gestalten oder Festigen der Frisur, bei dem man eine nicht waschaktive, wässrige, flüssige Zusammensetzung, die in einem kosmetisch unbedenklichen Medium
• mindestens ein Gellangummi oder ein Derivat davon,
• mindestens eine feste Verbindung, die aus Agar-Agar-Kugeln ausgewählt ist, in einer Konzentration zwischen 0,001 und 5 Gew.-%, bezogen auf das Gesamtgewicht der Zusammensetzung,
• und mindestens ein einwertiges Salz, das aus den Salzen einwertiger Kationen ausgewählt ist,
enthält, wobei die nicht waschaktive Zusammensetzung nicht mehr als 4 Gew.-% Tenside, bezogen auf das Gesamtgewicht der Zusammensetzung, enthält, verwendet.
